# EUROPÄISCHE PATENTSCHRIFT

(11) **EP 1 711 115 B1**
(45) Veröffentlichungstag und Bekanntmachung des Hinweises auf die Patenterteilung: **30.06.2010**
(21) Anmeldenummer: 04708700.2
(22) Anmeldetag: 06.02.2004
(51) Int. Cl.: A61B 17/88, A61F 2/46, A61C 5/00, B05C 17/01

(54) **EINSPRITZVORRICHTUNG, INSBESONDERE FÜR KNOCHENZEMENTE**
INJECTION DEVICE, ESPECIALLY FOR BONE CEMENT
DISPOSITIF D'INJECTION, EN PARTICULIER POUR CIMENT OSSEUX

(43) Veröffentlichungstag der Anmeldung: 18.10.2006
(73) Patentinhaber: Dr.h.c. Robert Mathys Stiftung, 2544 Bettlach (CH)
(72) Erfinder: BOHNER, Marc, 2544 Grenchen (CH); HEINI, Paul, CH-3084 Wabern (CH)
(74) Vertreter: Lusuardi, Werther
(86) Internationale Anmeldenummer: PCT/CH2004/000067
(87) Internationale Veröffentlichungsnummer: WO 2005/074840

(56) Entgegenhaltungen:
- EP-A- 1 219 263
- WO-A-00/23002
- WO-A-01/52924
- US-A- 2 903 794
- US-A- 4 338 925
- US-A- 4 993 948

## Beschreibung

Die Erfindung bezieht sich auf eine Einspritzvorrichtung gemäss dem Oberbegriff des Patentanspruchs 1.

Einspritzvorrichtungen, welche insbesondere für frisch gemischte Knochenzemente eingesetzt werden, unterscheiden sich von solchen für Flüssigkeiten darin, dass die engste Durchtrittsöffnung für den einzuspritzenden Stoff kritisch ist und das Verhältnis zwischen dem Durchmesser der engsten Durchtrittsöffnung und dem Durchmesser im Hohlraum des Spritzenkörpers einen bestimmten Mindestwert nicht unterschreiten sollte. Bei Unterschreiten eines solchen Mindestwertes entstehen bei der Anwendung einer Einspritzvorrichtung in der Chirurgie, insbesondere an der Wirbelsäule zu hohe Einspritzkräfte. Hohe Einspritzkräfte erfordern eine stärkere Kraftanwendung durch den Chirurgen und eine höhere Resistenz gegen mechanische Brüche der aus Kunststoff bestehenden Einspritzvorrichtung. Der Chirurg könnte also - bei sehr hohen Einspritzkräften - möglicherweise nicht mehr in der Lage sein, den Zement einzuspritzen oder die Plastikspritze könnte brechen. Es besteht deshalb ein Bedürfnis Einspritzvorrichtungen mit tiefer Einspritzkraft bereitzustellen.

In der Wirbelsäulenchirurgie ist es extrem wichtig einen hochviskosen Knochenzement zu verwenden, weil dies die Gefahr reduziert, dass der Knochenzement in den Rückenmarkskanal ausfliessen kann. Ein hochviskoser Knochenzement führt jedoch zu hohen Einspritzkräften. Das Bedürfnis nach Einspritzvorrichtungen mit tiefer Einspritzkraft ist somit für die Wirbelsäulenchirurgie noch wichtiger, weil damit die mit dieser Technik verbundenen Risiken reduziert werden können.

Ein Spritze, welche mittels einer Luer-Lock Verbindung mit einer Kanüle verbindbar ist, ist beispielsweise aus der US 4,220,151 WHITNEY bekannt. Diese bekannte Luer-Lock Verbindung zeichnet sich dadurch aus, dass zwei konzentrisch ineinander angeordnete Hülsen am vorderen Ende der Spritze angebracht sind. Die äussere Hülse ist mit einem Innengewinde versehen, worin das hintere Ende der Kanüle einschraubbar ist. Die innere Hülse weist aussen einen Konus auf, welcher mit der komplementär konisch ausgestalteten Zentralbohrung am hinteren Ende der Kanüle beim Einschrauben der Kanüle in das Innengewinde eine luftdichte Konusverbindung bildet. Nachteilig an dieser bekannten Vorrichtung ist, dass durch diese innere Hülse der maximale Durchmesser der Zentralbohrung am Übergang zwischen Spritze und Kanute erheblich eingeschränkt wird.

Aus der US 4,993,948 [CAMERON et al.] ist eine Einspritzvorrichtung für Zahnfüllungsmaterial bekannt, welche ein Übergangssegment mit einer Bohrung aufweist, die am Übergang zwischen dem Hohlraum des Spritzenkörpers und der Bohrung des Anschlussstückes einen größeren Querschnitt als das Lumen der Kanüle an deren hinterem Ende aufweist.

Aus der US 4,338,925 [MILLER] ist eine weitere Einspritzvorrichtung für Knochenzement bekannt, welche eine Kanüle mit konstantem Querschnitt umfasst.

Schliesslich ist aus der WO 01/52924 [ULTRADENT PRODUCTS] eine Spritze bekannt, die mit einem Luer-Lock-Anschluss ausgestattet ist und zum Mischen und Verabreichen einer viskosen Masse geeignet ist.

Der Erfindung liegt die Aufgabe zugrunde, eine Einspritzvorrichtung zu schafen, welche ein Anschlussstück für eine Kanüle umfasst, wobei die engste Durchgangsöffnung für den einzuspritzenden Stoff am Übergang zwischen Anschlussstück und Kanüle möglichst gross ist und einen kritischen Wert nicht unterschreitet.

Die Erfindung löst die gestellte Aufgabe mit einer Einspritzvorrichtung, welche die Merkmale des Anspruchs 1 aufweist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Einspritzvorrichtung:
- ein relativ zum Durchmesser des Hohlraumes im Spritzenkörper grosser Durchmesser für die Austrittsöffnung und die Zentralbohrung der Kanüle erreichbar ist;
- eine erhebliche Reduktion der Einspritzkräfte erreichbar ist;
- ein geringerer Druckabbau möglich ist im Vergleich zum Stand der Technik;
- eine Anwendung für Knochenzemente im Bereich der Wirbelsäule möglich ist; und
- aufgrund dessen, dass die Zentralbohrung der Kanüle in axialer Richtung eine konstante Querschnittsfläche q aufweist, die Einspritzkräfte nicht durch Verengungen erhöht werden.

In wiederum einer anderen Ausführungsform weist der Hohlraum eine zur Längsachse orthogonale Querschnittsfläche Q > q auf, wobei das Verhältnis zwischen den Querschnittsflächen q : Q zwischen 0,2 und 0,05 liegt.

Durch die Wahl eines geeigneten Verhältnisses der Querschnittsflächen Q und q lässt sich die Verbindung zwischen Spritzenkörper und Kanüle insbesondere bezüglich der Einspritzkräfte optimal an die Eigenschaften des einzuspritzenden Materials anpassen. Bei hochviskosen Knochenzementen wird vorzugsweise ein grosses Verhältnis q : Q gewählt.

In einer weiteren Ausführungsform weist die Bohrung im Anschlussstück ein Innengewinde auf, welches beispielsweise als Sägezahngewinde ausgebildet sein kann. Die Kanüle weist an ihrem hinteren Ende Mittel zum Einschrauben in das Innengewinde auf, welche beispielsweise als Nocken oder als komplementäres Aussengewinde ausgebildet sein können. Dadurch sind die Verbindungselemente zwischen Spritzenkörper und Kanüle einfach herstellbar.

In wiederum einer weiteren Ausführungsform ist das Anschlussstück als Luer-Lock Verbindungsstück ohne inneres Konuselement ausgebildet ist. Damit ist der Vorteil erreichbar, dass eine handelsübliche Kanüle mit einem Luer-Lock Verbindungsstück mit dem Anschlussstück des Spritzenkörpers verbindbar ist. Vorzugsweise entsprechen der Durchmesser der Bohrung im Übergangssegment zwischen Hohlraum und Anschlussstück und die Geometrie des Innengewindes im Anschlussstück den standardisierten Abmessungen einer Luer-Lock Verbindung. Die Mittel an der Kanüle zum Einschrauben in das Innengewinde können komplett als Luer-Lock Verbindungsstück ausgebildet sein.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Längsschnitt durch eine Ausführungsform der erfindungsgemässen Einspritzvorrichtung; und
Fig. 2 eine Schnitt längs der Linie II-II in Fig. 1.

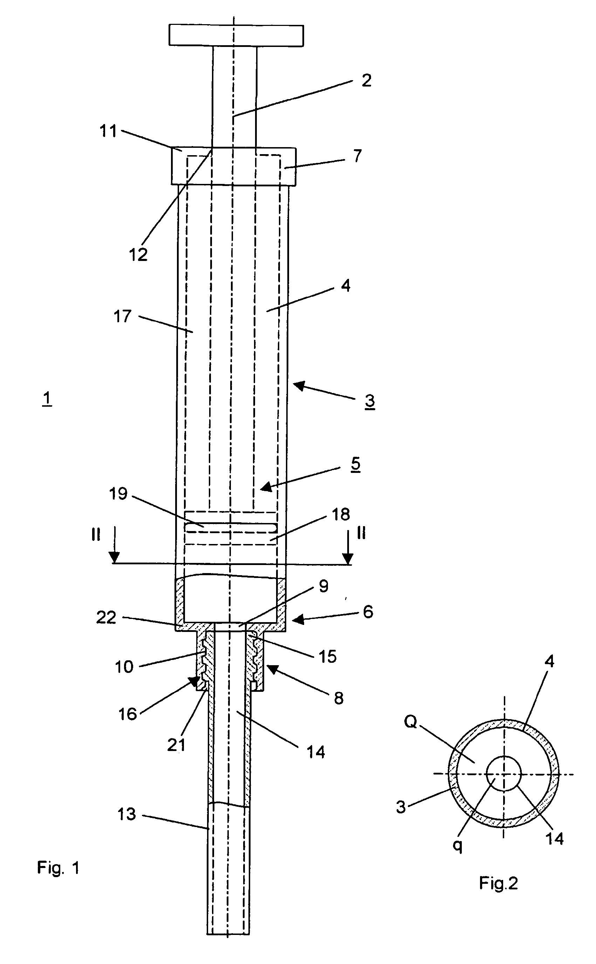

In den Fig. 1 und 2 ist eine Ausführungsform der Einspritzvorrichtung 1 dargestellt, welche einen zu einer Längsachse 2 koaxialen Spritzenkörper 3, einen im Hohlraum 4 des Spritzenkörpers 3 axial verschiebbaren Einspritzkolben 5 und eine am vorderen Ende 6 des Spritzenkörper 3 lösbar befestigbare Kanüle 13 umfasst. Der Spritzenkörper 3 ist an seinem hinteren Ende 7 mittels einer eine koaxial durchgehende Öffnung 12 aufweisenden Verschlusskappe 11 verschliessbar. Der Durchmesser der Öffnung 12 ist so gewählt, dass die Kolbenstange 17 des Einspritzkolbens 5 in der Öffnung 12 axial verschiebbar gelagert ist. Ferner umfasst der Einspritzkolben 5 einen am vorderen Ende 14 der Kolbenstange 13 befestigten Kolben 18, welcher peripher ein Dichtelement 19 aufweist, wodurch der Zwischenraum zwischen dem Kolben 18 und der Wand 20 des Hohlraumes 4 abgedichtet wird. Die Dichtelemente können beispielsweise aus Viton, Polypropylen, Poylbutyl oder Teflon bestehen.

An seinem vorderen Ende 6 weist der Spritzenkörper 3 ein in das Anschlussstück 8 mündendes Übergangssegment 22 mit einer koaxialen Bohrung 9 auf. Das Anschlussstück 8 ist mit einer zur Längsachse 2 koaxialen Bohrung 21 versehen. Die Bohrung 21 des Anschlussstückes 8 ist mit einem Innengewinde 10 versehen. Die Kanüle 13 ist an ihrem hinteren Ende 15 mit Mitteln 16 zum Einschrauben in das Innengewinde 10 versehen, welche hier als zum Innengewinde 10 komplementäres Aussengewinde ausgestaltet sind. Die zur Längsachse 2 koaxiale Zentralbohrung 14 der Kanüle 13 ist hier kreiszylindrisch mit einem Durchmesser d ausgebildet und weist eine zur Längsachse 3 orthogonale Querschnittsfläche q auf, während der Hohlraum 4 des Spritzenkörpers 3 eine zur Längsachse 3 orthogonale Querschnittsfläche Q > q aufweist. Ferner ist die Bohrung 9, welche am Übergangssegment 22 koaxial angeordnet ist und den Hohlraum 4 mit der Zentralbohrung 14 verbindet, ebenfalls kreiszylindrisch mit dem gleichen Durchmesser d ausgestattet, so dass die Zentralbohrung 14 am hinteren Ende 15 der Kanüle 13 mit der Bohrung 9 im Übergangssegment 22 am vorderen Ende 6 des Spritzenkörpers 3 fluchtend ausgebildet ist.

## Patentansprüche

1. Einspritzvorrichtung (1), insbesondere für Knochenzement, umfassend
A) einen Spritzenkörper (3) mit einer Längsachse (2), einem vorderen Ende (6), einem am vorderen Ende (6) angeordneten, eine koaxiale Bohrung (21) aufweisenden Anschlussstück (8) und einem koaxialen Hohlraum (4);
B) einen im Hohlraum (4) koaxial verschiebbaren Einspritzkolben (5); und
C) eine mit dem Anschlussstück (8) verbindbare Kanüle (13) mit einer Zentralbohrung (14) und einem hinteren Ende (15), wobei
D) der Spritzenkörper (3) an seinem vorderen Ende (6) ein Übergangssegment (22) mit einer koaxialen, den Hohlraum (4) mit der Bohrung (21) im Anschlussstück (8) verbindende Bohrung (9) mit konstantem Durchmesser aufweist;
E) die Bohrung (9) im Übergangssegment (22) und die Zentralbohrung (14) mindestens am hinteren Ende (15) der Kanüle (13) dieselbe zur Längsachse (2) orthogonale Querschnittsfläche q aufweisen; **dadurch gekennzeichnet, dass**
F) die Zentralbohrung (14) der Kanüle (13) in axialer Richtung eine konstante Querschnittsfläche q aufweist;
G) der Hohlraum (4) eine zur Längsachse (2) orthogonale Querschnittsfläche Q aufweist und dass das Verhältnis zwischen den Querschnittsflächen q : Q zwischen 0,200 und 0,033 liegt; und
H) das Anschlussstück (8) als Luer-Lock Verbindungsstück ohne inneres Konuselement ausgebildet ist.

2. Einspritzvorrichtung (1) nach Anspruch 1, **dadurch gekennzeichnet, dass** der Hohlraum (4) eine zur Längsachse (2) orthogonale Querschnittsfläche Q aufweist und dass das Verhältnis zwischen den Querschnittsflächen q : Q zwischen 0,2 und 0,05 liegt.

3. Einspritzvorrichtung (1) nach Anspruch 1 oder 2, **dadurch gekennzeichnet, dass** die Bohrung (9) im Anschlussstück (8) ein Innengewinde (10) aufweist.

4. Einspritzvorrichtung (1) nach Anspruch 3, **dadurch gekennzeichnet, dass** die Kanüle (13) am hinteren Ende (15) Mittel (16) zum Einschrauben in das Innengewinde (10) umfasst.

5. Einspritzvorrichtung (1) nach Anspruch 4, **dadurch gekennzeichnet, dass** die Mittel (16) ein zum Innengewinde (10) komplementäres Aussengewinde sind.

6. Einspritzvorrichtung (1) nach einem der Ansprüche 3 bis 5, **dadurch gekennzeichnet, dass** der Durchmesser der Bohrung (9) im Übergangssegment (22) und die Geometrie des Innengewindes (10) im Anschlussstück (8) denjenigen einer Luer-Lock Verbindung entsprechen.

7. Einspritzvorrichtung (1) nach einem der Ansprüche 4 bis 6, **dadurch gekennzeichnet, dass** die Mittel (16) zum Einschrauben in das Innengewinde (10) als Luer-Lock Verbindungsstück ausgebildet sind.

## Claims

1. An injection device (1), especially for bone cement, comprising
A) an injection body (3) with a longitudinal axis (2), a front end (6), a connecting piece (8), which is disposed at the front end (6) and has a coaxial borehole (21), and a coaxial cavity (4)
B) an injection piston (5), which can be shifted coaxially in the cavity (4); and
C) a cannula (13), which can be connected with the connecting piece (8), has a central borehole (14) and a rear end (15), wherein
D) at its front and (6), the injection body (3) has a transition segment (22) with a coaxial borehole (9) of constant diameter, which connects the cavity (4) with the borehole 21 in the connecting piece (8)
E) the borehole (9) in the transition segment (22) and the central borehole (14), at least at the rear end (15) of the cannula (13), have the same cross-sectional area q, orthogonal to the longitudinal axis (2);
**characterized in that**
F) the central borehole (14) of the cannula (13) has a constant cross-sectional area q in the axial direction;
G) the cavity (4) has a cross-sectional area Q orthogonal to the longitudinal direction (2) and that the ratio between the cross-sectional areas q and Q is between 0.200 and 0.033 and
H) the connecting piece (8) is constructed as a Luer Lock connecting piece without an internal conical element.

2. The injection device (1) of claim 1, **characterized in that** the cavity (4) has a cross sectional area Q orthogonal to the longitudinal axis (2) and that the ratio of the cross-sectional area q to the cross-sectional area Q is between 0.2 and 0.05.

3. The injection device (1) of claims 1 or 2, **characterized in that** the borehole (9) has an internal thread (10) in the connecting piece (8).

4. The injection device (1) of claim 3, **characterized in that,** at the rear end (15), the cannula (13) comprises means (16) for screwing into the internal thread (10).

5. The injection device (1) of claim 4, **characterized in that** the means (16) are an external thread, which is complementary to the internal thread (10).

6. The injection device (1) of one of the claims 3 can 5, **characterized in that** the diameter of the borehole (9) in the transition segment (22) and the geometry of the internal thread (10) in the connection piece (8) correspond to those of a Luer Lock connection.

7. The injection device (1) of one of the claims 4 to 6, **characterized in that** the means (16) for screwing in the internal thread (10) are constructed as a Luer Lock connecting piece.

## Revendications

1. Dispositif d'injection (1), en particulier pour ciment osseux, comprenant :
A) un corps de seringue (3) présentant un axe longitudinal (2), une extrémité antérieure (6), une pièce de raccordement (8) située au niveau de l'extrémité antérieure (6) possédant un alésage coaxial (21), et un espace creux coaxial (4) ;
B) un piston d'injection (5) déplaçable coaxialement dans l'espace creux (4) ; et
C) une canule (13) pouvant être reliée à la pièce de raccordement (8), laquelle canule présente un alésage central (14) et une extrémité arrière (15), dans lequel
D) le corps de seringue (3) présente, au niveau de son extrémité antérieure (6), un segment de transition (22) ayant un alésage coaxial (9) à diamètre constant reliant l'espace creux (4) à l'alésage (21) situé dans la pièce de raccordement (8) ;
E) l'alésage (9) dans le segment de transition (22) et l'alésage central (14) qui présentent, au moins au niveau de l'extrémité arrière (15) de la canule (13), la même aire de section droite q orthogonale à l'axe longitudinal (2) ;
**caractérisé en ce que**
F) l'alésage central (14) de la canule (13) présente une aire de section droite q constante dans le sens axial ;
G) l'espace creux (4) présente une aire de section droite Q orthogonale à l'axe longitudinal (2) et le rapport des aires de section droite q:Q est compris entre 0,200 et 0,033 ; et
H) la pièce de raccordement (8) est réalisée sous la forme d'un raccord Luer-Lock sans élément conique interne.

2. Dispositif d'injection (1) selon la revendication 1, **caractérisé en ce que** l'espace creux (4) présente une aire de section droite Q orthogonale à l'axe longitudinal (2) et le rapport des aires de section droite q:Q est compris entre 0,2 et 0,05.

3. Dispositif d'injection (1) selon la revendication 1 ou 2, **caractérisé en ce que** l'alésage (9) situé dans la pièce de raccordement (8) présente un taraudage (10).

4. Dispositif d'injection (1) selon la revendication 3, **caractérisé en ce que** la canule (13) comprend, au niveau de l'extrémité arrière (15), des moyens (16) permettant un vissage dans le taraudage (10).

5. Dispositif d'injection (1) selon la revendication 4, **caractérisé en ce que** les moyens (16) sont réalisés sous la forme d'un filetage complémentaire au taraudage (10).

6. Dispositif d'injection (1) selon l'une quelconque des revendications 3 à 5, **caractérisé en ce que** le diamètre de l'alésage (9) dans le segment de transition (22) et la géométrie du taraudage (10) dans la pièce de raccordement (8) correspondent à ceux d'un raccord Luer-Lock.

7. Dispositif d'injection (1) selon l'une quelconque des revendications 4 à 6, **caractérisé en ce que** les moyens (16) pour le vissage dans le taraudage (10) sont réalisés sous la forme d'un raccord Luer-Lock.
